Europäisches Patentamt

European Patent Office

Office européen des brevets

(19)

(11) **EP 1 062 966 B1**

(12) **EUROPEAN PATENT SPECIFICATION**

(45) Date of publication and mention
of the grant of the patent:
**01.09.2004   Bulletin 2004/36**

(51) Int Cl.$^7$: **A61M 25/10**

(21) Application number: **00113074.9**

(22) Date of filing: **26.06.2000**

(54) **Balloon for dilating a stricture and balloon catheter**

Ballon zum Aufweiten einer Verengung und Ballonkatheter

Ballonnet pour dilater un rétrécissement et cathéter à ballonnet

(84) Designated Contracting States:
**AT BE CH CY DE DK ES FI FR GB GR IE IT LI LU
MC NL PT SE**

(30) Priority: **25.06.1999   JP 18077299**

(43) Date of publication of application:
**27.12.2000   Bulletin 2000/52**

(73) Proprietor: **TERUMO KABUSHIKI KAISHA
Tokyo 151 (JP)**

(72) Inventors:
• **Yashiro, Naofumi,
Estate-Higashi-Shinkoiwa 6-307
Tokyo (JP)**
• **Ookushi, Naohisa, Terumo Kabushiki Kaisha
Shizuoka (JP)**

(74) Representative: **Casalonga, Axel et al
BUREAU D.A. CASALONGA - JOSSE
Paul-Heyse-Strasse 33
80336 München (DE)**

(56) References cited:
WO-A-97/40877        US-A- 5 055 024
US-A- 5 352 199      US-A- 5 415 635
US-A- 5 695 498      US-A- 5 749 851
US-A- 5 843 116

**Description**

The present invention relates to a balloon for dilating a stricture (stenosis), and to a balloon catheter including the same. Such a ballon according to the preamble of claim 1 has been described in US-A-5 415 635.

In percutaneous transluminal angioplasty (PTA) or percutaneous transluminal coronary angioplasty (PTCA), for example, a balloon catheter including a balloon that can be inflated and deflated is used.

This balloon catheter is inserted into the blood vessel from the outside of the body and inflates a balloon to dilate a stricture when the balloon reaches the stricture in the blood vessel that is a target region.

Most of the existing balloons used for this balloon catheter are shaped like a spindle, that is, have a fixed outer diameter when they are inflated. Therefore, when they are inflated to apply pressure to the stricture from the inside of the blood vessel, they may move in a front or a rear direction (distal end or proximal end direction) by reaction force from the stricture and deviate from the stricture.

When this deviation occurs, the balloon must be deflated, located at a proper position (the position of the stricture) and inflated again, or the balloon catheter must be exchanged with another balloon catheter having a different balloon size, thereby taking a lot of time and labor, hindering smooth treatment and increasing the burden of a patient.

Particularly when there is a difference in the diameter of the blood vessel between front and rear portions of the stricture or when the blood vessel curves at an acute angle or branches off as in the case where a stricture which is produced near a shunt inosculating portion formed by inosculating the cephalic vein with the radial artery for the dialysis of the blood is dilated by a balloon, the above deviation of the balloon from the stricture easily occurs and the balloon cannot be held at the target region stably during treatment.

In order to prevent the balloon from deviating from the stricture, it is conceivable to reduce the inflation pressure of the balloon. In this case, the stricture cannot be dilated to the full and a desired treatment effect cannot be expected.

USP 4,327,736 discloses a rubber balloon, which has a fixed outer diameter over the entire length and is fixed by winding a rubber band round the center portion to prevent the deviation of the balloon from a stricture. While the balloon is being inflated, the rubber band suppresses the expanding of the center portion of the balloon so that a depression is formed in the center portion. However, this balloon is shaped like a spindle as described above and the stricture is dilated while the expanding of the center portion is suppressed with the rubber band. Therefore, the expanded diameter of the balloon changes according to a slight change in inside pressure to be applied to the balloon, thereby making it difficult to control the expanded diameter.

It is an object of the present invention to provide a balloon for dilating a stricture, which is aligned with the stricture without fail when it is inflated and which can carry out the proper, safe and smooth PTA of the stricture as well as a balloon catheter including the same.

The above object of the present invention is attained by a ballon according to the present invention as defined in claim 1. The balloon according to the present invention may further comprise anyone of the following features (1) to (16).

(1) A balloon for dilating a stricture which can be inflated and deflated by a change in inside pressure and has a dilation function portion having the function of contacting tightly to the stricture of a lumen and dilating the stricture by pressure, and a first fixing portion and a second fixing portion located on proximal end and distal end sides of the dilation function portion, respectively, and having the function of fixing the balloon to the lumen, wherein

when the minimum outer diameter of the dilation function portion under first dilation pressure at which the dilation function portion dilates to a predetermined diameter is represented by $D_0$, the maximum outer diameter of the first fixing portion is represented by $D_1$ and the maximum outer diameter of the second fixing portion is represented by $D_2$, the balloon is molded to satisfy $D_0 < D_1$ and $D_0 < D_2$.

(2) The balloon for dilating a stricture according to (1) which satisfies $D_0\text{max} - D_0 \leq 0.15D_0$ when the minimum outer diameter of the dilation function portion under second dilation pressure at which the balloon ruptures by a rise in inside pressure is represented by $D_0\text{max}$.

(3) The balloon for dilating a stricture according to the above item (1) or (2) which satisfies $D_1 > D_2$ under the first dilation pressure.

(4) The balloon for dilating a stricture according to the above item (1) or (2) which satisfies $D_1 < D_2$ under the first dilation pressure.

(5) The balloon for dilating a stricture according to any one of the above items (1) to (4) which satisfies $0.02D_0 \leq \Delta D \leq 0.4D_0$ when $\Delta D = (D_1 + D_2)/2 - D_0$.

(6) The balloon for dilating a stricture according to any one of the above items (1) to (5), wherein $\Delta D$ is 0.02 to 20 mm when $\Delta D = (D_1 + D_2)/2 - D_0$.

(7) The balloon for dilating a stricture according to any one of the above items (1) to (6), wherein $L_0$ is 5 to 50 mm when the length in an axial direction of the dilation function portion under the first dilation pressure is represented by $L_0$.

(8) The balloon for dilating a stricture according to any one of the above items (1) to (7) which satisfies $L_1 > L_2$ when the length in an axial direction of the first fixing portion is represented by $L_1$ and the length in an axial direction of the second fixing portion is represented by $L_2$ under the first dilation pressure.

(9) The balloon for dilating a stricture according to any one of the above items (1) to (7) which satisfies $L_1 < L_2$ when the length in an axial direction of the first fixing portion is represented by $L_1$ and the length in an axial direction of the second fixing portion is represented by $L_2$ under the first dilation pressure.

(10) The balloon for dilating a stricture according to any one of the above items (1) to (9) which is produced by biaxial orientation and blow molding.

(11) The balloon for dilating a stricture according to any one of the above items (1) to (10), wherein the first fixing portion and/or the second fixing portion have/has higher pressure resistance than the dilation function portion.

(12) A balloon catheter comprising a catheter body having flexibility and the balloon for dilating a stricture according to any one of the above items (1) to (11) at the end portion of the catheter body.

(13) The balloon catheter according to the above item (12), wherein a lumen which is the passage of a working fluid for inflating and deflating the balloon for dilating a stricture and a lumen for containing a guide wire are formed in the catheter body.

(14) The balloon catheter according to the above item (12) or

(13) which comprises a marker for confirming the position of the balloon for dilating a stricture in the body.

(15) The balloon catheter according to the above item (14), wherein at least the position of the dilation function portion can be confirmed with the marker.

(16) The balloon catheter according to any one of the above items (12) to (15), wherein the catheter body projects from the distal end of the balloon for dilating a stricture by a predetermined length and the projecting portion bends at least one time.

(17) The balloon catheter according to the above items (1) to (16), wherein the dilation function portion has a constant predetermined outer diameter along its axial length.

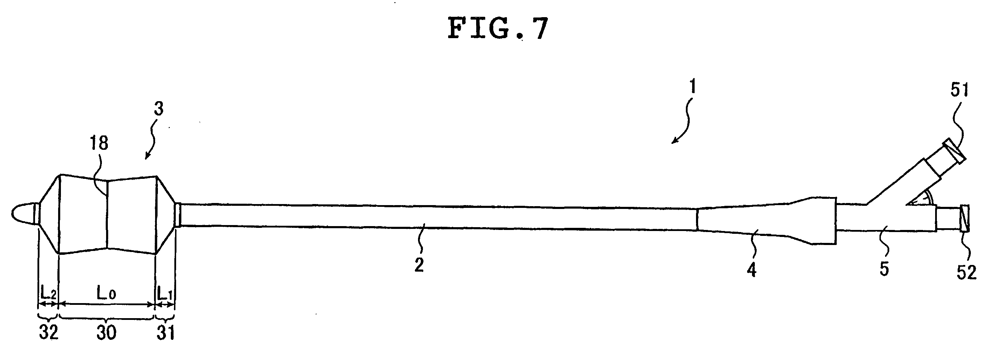

(18) The balloon catheter according to the above items (1) to (16), wherein the dilation function portion [30] has a minimum outer diameter in the center [18] of its axial length, and the outer diameter continuously increases toward both of the first [31] and the second [32] fixing portion (see Fig. 7).

These and other objects and advantages of the present invention will become clear from the following description with reference to the accompanying drawings, wherein:

Fig. 1 is an entire view of an embodiment of a balloon catheter comprising a balloon for dilating a stricture of the present invention;
Fig. 2 is a partial plan view of the balloon of another embodiment of a balloon catheter comprising the balloon for dilating a stricture of the present invention;
Fig. 3 is a partial plan view of the balloon of another embodiment of a balloon catheter comprising the balloon for dilating a stricture of the present invention;
Fig. 4 is a partial plan view of the balloon of another embodiment of a balloon catheter comprising the balloon for dilating a stricture of the present invention;
Fig. 5 is a partial plan view of the balloon of another embodiment of a balloon catheter comprising the balloon for dilating a stricture of the present invention;
Fig. 6 is a partial plan view of the balloon of another embodiment of a balloon catheter comprising the balloon for dilating a stricture of the present invention;
Fig. 7 is an entire view of the balloon of another embodiment of a balloon catheter comprising the balloon for dilating a stricture of the present invention;
Fig. 8 is a sectional view cut on line A-A of Fig. 1;
Fig. 9 is a sectional view cut on line B-B of Fig. 1;
Fig. 10 is a sectional view showing another embodiment of a catheter body;
Fig. 11 is a graph showing the relationship between the inside pressure of the balloon and the outer diameter of the balloon;
Fig. 12 is a diagram schematically showing the use state (when the balloon is deflated) of the balloon catheter of the present invention; and
Fig. 13 is a diagram schematically showing the use state (when the balloon is inflated) of the balloon catheter of the present invention.

The balloon for dilating a stricture and the balloon catheter of the present invention will be described in detail hereinafter with reference to preferred embodiments shown in the accompanying drawings.

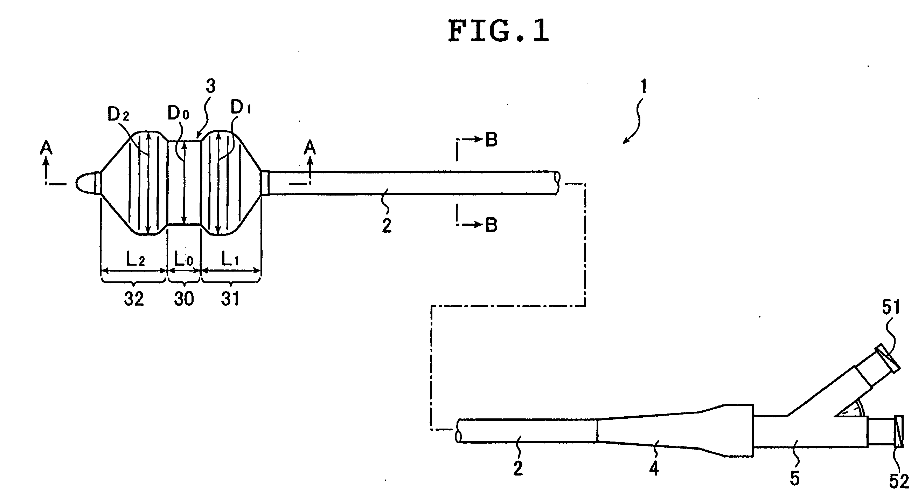

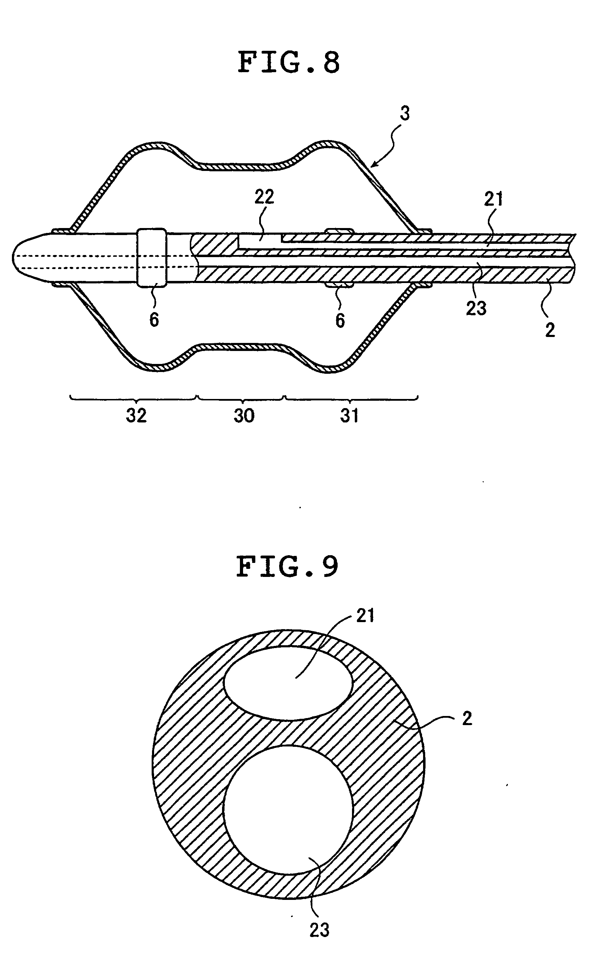

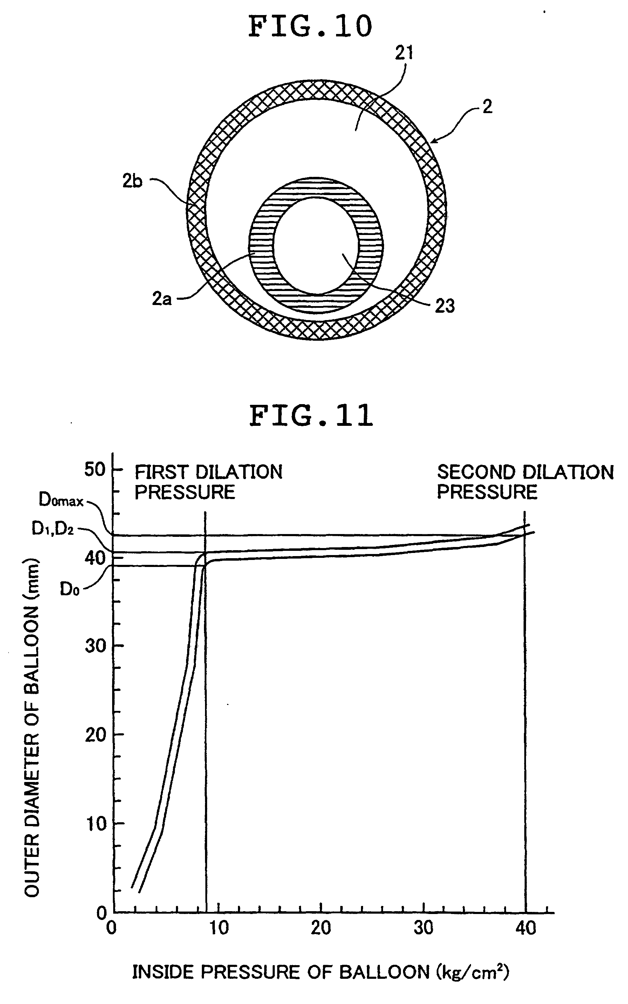

Fig. 1 is an entire view of an embodiment of a balloon catheter comprising the balloon for dilating a stricture of the present invention (to be simply referred to as "balloon" hereinafter), Fig. 8 is a sectional view cut on line A-A of Fig. 1 and Fig. 9 is a sectional view cut on line B-B of Fig. 1. In the following description, the right sides of Fig. 1 and Fig. 8 are illustrated as "proximal end" and the left sides as "distal end".

As shown in Fig. 1, Fig. 8 and Fig. 9, the balloon catheter 1 of the present invention has a catheter body 2, a balloon 3 attached to the periphery of the distal end portion of the catheter body 2 and a hub 5 attached to the proximal end portion of the catheter body 2 by a connection tube 4.

The catheter body 2 has desired flexibility and may be made from a thermoplastic resin such as a polyolefin-based resin, polyamide-based resin, urethane-based resin or polyimide-based resin, or a thermosetting resin. Illustrative examples of the material include polyolefins such as polyethylene, polypropylene, ethylene-propylene copolymer and ethylene-vinyl acetate copolymer (EVA), polyesters such as polyvinyl chloride, polyethylene terephthalate (PET) and polybutylene terephthalate (PBT), and polyurethane-, polyamide-, polyimide- and polystyrene-based resins, fluororesin, styrene-, polyolefin-, polyvinyl chloride-, polyurethane, polyester-, polyamide, polybutadiene- and fluororubber-based thermoplastic elastomers.

The catheter body 2 may have a multi-layer laminate structure made from a plurality of materials.

A lumen 21 which is the passage of a working fluid for inflating and deflating the balloon 3 and a lumen 23 for containing a guide wire (not shown) are formed in the catheter body 2.

As shown in Fig. 8, a side hole 22 is formed in the outer surface of the catheter body 2 internal to the balloon 3 and the distal end of the lumen 21 communicates with the side hole 22. Thus, the lumen 21 communicates with the inside of the balloon 3.

The distal end of the lumen 23 is open to the distal end of the catheter body 2. This lumen 23 is used to insert the guide wire and also to supply a medical liquid etc. into the body or suck a liquid.

As shown in Fig. 9, the shapes of the sections of the lumens 21 and 23 are circular or oval but are not limited to these. For example, the section of the lumen 21 may be shaped like a crescent in accordance with the circumference of the lumen 23. A lumen other than the lumens 21 and 23 may be existent.

The dimensions (outer diameter, length, etc.) of the catheter body 2 are not particularly limited but are suitably determined according to the application purpose of the balloon catheter 1 and the case of a disease, etc. The outer diameter of the catheter body 2 is preferably about 0.7 to 3.0 mm and the length thereof is preferably about 30 to 150 cm.

The hub 5 attached to the proximal end portion of the catheter body 2 is forked and ports 51 and 52 are formed in each end of branches. The port 51 communicates with the lumen 21 of the catheter body 2 and the port 52 communicates with the lumen 23.

The guide wire is inserted into the lumen 23 from the port 52 to locate the distal end thereof at a position near the distal end of the lumen 23 so as to perform guiding the distal end in case of insertion of the balloon catheter 1 into the blood vessel (lumen).

A working fluid injector such as a syringe is connected to the port 51 and operated to supply the working fluid (for example, liquid such as physiological saline, and an X-ray contrast media, air or gas such as air and carbonic acid gas) into the balloon 3 through the lumen 21 to inflate the balloon 3.

The material of the hub 5 is not particularly limited and may be a resin material such as polyvinyl chloride, polyethylene, polypropylene, polycarbonate, polymethyl methacrylate and acrylonitrile-styrene-butadiene copolymer or a metal material.

The balloon 3 is made of a cylindrical film whose proximal end portion and distal end portion are fixed to the outer surface of the catheter body 2 airtightly. The proximal end portion and distal end portion of the balloon 3 are fixed to the catheter body 2 by fusion or adhesion with an adhesive.

The catheter body 2 may be formed as shown in Fig. 10. The catheter body 2 shown in Fig. 10 comprises a coaxial tube including an inner tube 2a and an outer tube 2b provided outside the inner tube 2a coaxially. The lumen 23 for inserting the guide wire (not shown) is formed in the inner tube 2a, and the lumen 21 serving as the passage of the working fluid for inflating or deflating the balloon 3 is formed in the approximately annular space between the inner tube 2a and the outer tube 2b. In this case, the proximal end portion and the distal end portion of the balloon 3 are fixed airtightly to the distal end portion of the outer tube 2b and the distal end portion of the inner tube 2a, respectively.

The balloon 3 is preferably attached concentric or coaxially to the catheter body 2. As shown in the figure 1, the distal end of the catheter body 2 preferably projects from the distal end of the balloon 3 by a predetermined length. This projecting portion is preferably formed more flexible than other portions of the catheter body 2. Thereby, the inner wall of the blood vessel is prevented from being damaged with more certainty when the balloon catheter 1 is inserted into the blood vessel.

The balloon 3 has a dilation function portion 30 having the function of contacting tightly or closely to the stricture (lesion site) of a lumen (typified by "the blood vessel" hereinafter) such as the blood vessel and dilating the stricture by pressure (to be referred to as "stricture dilation function" hereinafter) and a first fixing portion 31 and a second fixing portion 32 which are located on the proximal end side and distal end side of the dilation function portion 30, respectively, and have the function of fixing the balloon 3 to the blood vessel (to be referred to as "balloon fixing function" hereinafter).

Although this balloon 3 is deflated and folded before inflation (at the time of shrinkage), when the working fluid is supplied into the inside of the balloon to inflate the balloon, it is formed into a shape which will be described below.

When the minimum outer diameter of the dilation function portion 30 under first dilation pressure at which the dilation function portion 30 dilates to a predetermined nominal diameter is represented by $D_0$, the maximum outer diameter of the first fixing portion 31 under the first dilation pressure is represented by $D_1$ and the maximum outer diameter of the second fixing portion 32 under the first dilation pressure is represented by $D_2$, they satisfy the expressions $D_0 < D_1$ and $D_0 < D_2$. Thus, the first fixing portion 31 and the second fixing portion 32 can fix the balloon 3 to the blood vessel at the both ends of the stricture without fail, the balloon 3 is prevented from being dislocated from the stricture, and the stricture can be dilated properly and surely.

In this embodiment, $D_1$ and $D_2$ are almost equal to each other.

When $\Delta D = (D_1 + D_2)/2 - D_0$, $\Delta D$ preferably satisfies the expression $0.02D_0 \leq \Delta D \leq 0.4D_0$, more preferably the expression $0.05D_0 \leq \Delta D \leq 0.3D_0$. When $\Delta D$ is smaller than $0.02D_0$, the difference of outer diameter between the dilation function portion 30 and the first fixing portion 31/the second fixing portion 32 is small and the balloon fixing function may not be fully exhibited according to other conditions. When $\Delta D$ is larger than $0.4D_0$, the difference of outer diameter between the dilation function portion 30 and the first fixing portion 31/the second fixing portion 32 is large and the stricture dilation function of the dilation function portion 30 may not be fully exhibited according to the shape, size and the like of the stricture and the operation of removing the balloon catheter 1 by deflating the balloon 3 again after inflation may be difficult.

$\Delta D$ is preferably about 0.02 to 20 mm, more preferably about 0.1 to 8 mm, much more preferably about 0.4 to 3.6 mm. When $\Delta D$ is smaller than 0.02 mm, the difference of outer diameter between the dilation function portion 30 and the first fixing portion 31/the second fixing portion 32 is small and the balloon fixing function may not be fully exhibited according to other conditions. When $\Delta D$ is larger than 20 mm, the difference of outer diameter between the dilation function portion 30 and the first fixing portion 31/the second fixing portion 32 is large and the stricture dilation function of the dilation function portion 30 may not be fully exhibited according to the shape, size and the like of the stricture and the operation of removing the balloon catheter 1 by deflating the balloon 3 again after inflation may be difficult.

The value of $D_0$ is not particularly limited and is suitably determined according to the application purpose of the balloon catheter 1 and the case of a disease. It is preferably about 1 to 30 mm, more preferably about 2 to 20 mm, much more preferably about 3 to 9 mm.

Fig. 11 is a graph showing an example of the relationship between the inside pressure of the balloon 3 and the outer diameter of the balloon 3 (the respective outer diameters of the dilation function portion 30 and the first fixing portion 31/the second fixing portion 32). It is needless to say that the curve of the graph changes according to the material, size and production conditions etc. of the balloon 3.

When the working fluid is injected into the balloon 3 to gradually raise the inside pressure of the balloon 3, the outer diameter of the balloon 3 gradually increases. When the inside pressure of the balloon 3 reaches the first dilation pressure at which the balloon 3 (dilation function portion 30) expands almost to a predetermined nominal diameter, the increase rate of the outer diameter of the balloon 3 sharply decreases, the outer diameter of the balloon 3 is rarely increased by further raising the inside pressure of the balloon 3 (little compliance), and the balloon 3 ruptures in the end. The inside pressure of the balloon 3 just before this rupture is represented by second dilation pressure.

The balloon 3 of the present invention preferably satisfies the expression $D_0\max - D_0 \leq 0.15D_0$, more preferably the expression $D_0\max - D_0 \leq 0.1D_0$ when the minimum outer diameter of the dilation function portion 30 under the second dilation pressure is represented by $D_0\max$. Thereby, even when the inside pressure of the balloon 3 exceeds the first dilation pressure, the balloon 3 is prevented from expanding excessively to dilate the stricture excessively, thereby making it possible to dilate the stricture properly and safely.

The length $L_0$ in the axial direction of the dilation function portion 30 under the first dilation pressure, the length $L_1$ in the axial direction of the first fixing portion 31 under the first dilation pressure and the length $L_2$ in the axial direction of the second fixing portion 32 under the first dilation pressure are not particularly limited but preferably are in the following ranges.

That is, $L_0$ is preferably in the range of about 5 to 50 mm, more preferably about 10 to 40 mm. Thus, fitness with the stricture is enhanced, the interval between the first fixing portion 31 and the second fixing portion 32 does not become too large, and both the stricture dilation function and the balloon fixing function can be exhibited more effectively.

$L_1$ and $L_2$ are preferably about 1 to 8 mm, more preferably about 2 to 4 mm. Further, it is preferable that the outer surfaces of the first fixing portion 31, the dilation function portion 30 and the second fixing portion 32 of the balloon 3 under the first dilation pressure are connected to one another by a continuous curved surface (smooth curved surface) in the axial direction, that is, a change in the outer diameter of the balloon 3 in the axial direction is continuous. Thus, an increase in the size of the balloon 3 is prevented and an excellent balloon fixing function can be exhibited. In this embodiment, $L_1$ and $L_2$ are almost equal to each other.

For example, the above size conditions are particularly preferred in a balloon catheter used in the PTA of a shunt formed by inosculating the radial artery with the cephalic vein or a shunt formed by inosculating the radial artery with the radial cutaneous vein.

Since the balloon 3 having the above characteristic can control the required expanded diameter easily, appropriate dilation pressure is applied to the stricture without fail and the inflated balloon 3 is fixed without fail and is not dislocated in front and rear directions, the PTA of the stricture can be carried out easily and properly, the time required for PTA can be shortened, and the burden of a patient is reduced.

The balloon 3 is made from various kinds of polymer material (particularly a thermoplastic resin). In this case, the entire balloon 3 is preferably made from a material having flexibility but relatively small elasticity (elongation).

Illustrative examples of the material of the balloon 3 include polyester resins such as polyethylene terephthalate, polyethylene naphthalate, polybutylene terephthalate and polybutylene naphthalate, polyester elastomers containing these, olefin-based resins such as polyethylene and polypropylene, crosslinked products thereof (particularly crosslinked by exposure to electron beams), polyamide-based resins such as nylon 11, nylon 12 and nylon 610, polyamide elastomers containing these, polyurethane resins, ethylene-vinyl acetate copolymer, crosslinked products thereof, polymer blends containing at least one of these, polymer alloys and the like.

Further, the balloon 3 may be made from a laminate consisting of a plurality of films made from these materials. In this case, the laminate can be obtained by, for example, coextruding and forming each layer and bonding these layers by adhesion or fusion, or forming other layers on one layer by coating. A soft resin layer, lubricating material layer, anti-thrombic material layer, or the like may be formed on the exterior side or interior side of the laminate made from the above materials.

The material of the balloon 3 may contain an anti-thrombic material such as heparin, prostaglandin, urokinase or arginine derivative and an X-ray impermeable material such as barium sulfate, barium carbonate, bismuth oxide or tungsten which functions as a marker to be described hereinafter. The X-ray impermeable material may be existent on the dilation function portion 30 alone (for instance, a layer containing an X-ray impermeable material is formed only on the interior side of the dilation function portion 30).

The above balloon 3 can be produced by biaxial orientation and blow molding as will be described hereinafter.

A tube (cylinder) made from the above material is stretched to a predetermined length at an appropriate temperature (for example, 150 to 300°C). Thus, the tube is stretched in an axial direction (longitudinal direction of the catheter body 2).

Next, the stretched tube is inflated in a metal mold and blow molded. The molding space (cavity) of the metal mold is made almost the same shape as the shape of the inflated balloon 3. In the metal mold, high-pressure gas such as nitrogen gas is injected into the tube. For blow molding, the temperature of the tube is raised by heating the metal mold to soften the tube and inflate it in a radial direction. Thus, the tube is stretched in a direction different from the axial direction of the first stretching with the result that the balloon 3 is obtained by biaxial orientation. The stretching in the axial direction of the tube may be carried out after blow molding.

The balloon 3 having high dimensional accuracy and small fluctuations in shape, film strength, characteristics, etc. can be produced easily by employing the above production process.

Particularly preferably, the above blow molding is carried out using a metal mold having a diameter smaller than the desired size, portions corresponding to a tapered portion on the proximal end side from the first fixing portion 31 (tapered portion between the first fixing portion 31 and a connection portion with the catheter body 2) and/or the second fixing portion 32 (tapered portion between the first fixing portion 31 and a connection portion with the catheter body 2) of the molded product are stretched in an axial direction at a temperature below the glass transition point of the resin to make the tapered portion thinner, and the molded product is set in a metal mold having a desired size and shape and is blow molded by heating and pressurizing. Thus, a balloon having a relatively thin tapered portion can be obtained and the deflated balloon can be folded or wound round the catheter body 2 in a compact manner.

The balloon 3 of the present invention can be produced by the above process. However, since the balloon 3 satisfies the conditions $D_0 < D_1$ and $D_0 < D_2$ as described above, the first fixing portion 31 and the second fixing portion 32 have a larger expansion coefficient (elongation) in a radial direction than the dilation function portion 30. Therefore, there develops a difference in strength between them, whereby the first fixing portion 31 and the second fixing portion 32 rupture more easily than the dilation function portion 30 at the time of molding and use (inflation) of balloon 3.

Therefore, it is preferred to make (reinforce) the pressure resistance of one or both of the first fixing portion 31 and the second fixing portion 32 higher than that of the dilation function portion 30. Thus, the damage and rupture of the balloon 3 can be prevented without fail. Particularly when the inflation and deflation of the balloon 3 are repeated several times, the damage and rupture of the balloon 3 can be prevented, and extremely high safety can be obtained.

To increase the pressure resistance, the thickness of the film of the first fixing portion 31 and/or the second fixing portion 32 is made larger than that of the dilation function portion 30. Describing this method in more detail, after the balloon is molded by the above biaxial orientation, blow molding, etc., such a phenomenon that the first fixing portion 31 and/or the second fixing portion 32 are/is heated at preferably a temperature near the secondary transition point of their material to shrink only the heated portions is used to partially change the thickness.

Also, the thickness of the film can be partially changed by varying the elongation ratios in a radial direction or axial direction of the first fixing portion 31 and/or the second fixing portion 32 from the ratios of the dilation function portion 30, or by varying the heating temperature for blow molding.

Another method of increasing the pressure resistance of the first fixing portion 31 and/or the second fixing portion 32 is to form the balloon 3 from a material which can be crosslinked by exposure of high energy beams (including X-rays, β-rays, heavily charged corpuscular rays, neutron rays, etc. in a special case) such as ultraviolet radiation, electron beams and γ-radiation and to selectively irradiate the first fixing portion 31 and/or the second fixing portion 32 with the above ultraviolet radiation or the like so as to crosslink only the portion(s) (or to make the degree of crosslinking of the portion(s) higher than the other portions). As the degree of crosslinking becomes higher, the strength of the film increases, and the pressure resistance improves. The selective irradiation of ultraviolet radiation or the like is made possible by masking portions not to be exposed to ultraviolet radiation or the like.

As means of making a difference in the degree of crosslinking, not only the selective irradiation of ultraviolet radiation or the like but also making a difference in heat energy (heating temperature, heating time) or the like may be used.

Still another method of improving the pressure resistance of the first fixing portion 31 and/or the second fixing portion 32 is to place a reinforcement on the first fixing portion 31 and/or the second fixing portion 32. The reinforcement may be a thin film formed from a hard material such as a metal on the interior surface or exterior surface of a portion to be reinforced of the film by deposition or the like.

The strength in a radial direction of the balloon 3 produced by the above method can be represented by the following expression:

$$\sigma_2 = p \cdot r \cdot h$$

wherein $\sigma_2$ is the calculated tensile strength of the film of the balloon 3, p is applied pressure inside the balloon, r is the radius of the balloon 3 and h is the thickness of the film.

As for the strength of the balloon 3 of the present invention, the calculated tensile strength is preferably 1,600 kg/cm$^2$ or more, more preferably 1,600 to 2,100 kg/cm$^2$ when the thickness of the film is about 0.01 to 0.05 mm and the second dilation pressure is 30 to 37 kg/cm$^2$.

As shown in Fig. 8, at least one marker 6 (a plurality of markers in this embodiment) for checking the position of the balloon 3 in the body by fluoroscopy is provided on the outer surface of the catheter body 2 in the balloon 3. This marker 6 is made from an X-ray impermeable material such as a metal exemplified by platinum, gold, silver, titanium or tungsten, or an alloy thereof.

In this embodiment, the marker 6 is shaped like a ring covering the outer surface of the catheter body 2. However, the marker 6 is not limited to this and may be shaped like a chip or a block.

The markers 6 are provided at positions corresponding to the first fixing portion 31 and the second fixing portion 32 of the balloon 3. Thus, the positions of the first fixing portion 30 and the second fixing portion 32 can be confirmed by fluoroscopy and the position of the dilation function portion 31 between them can be confirmed, thereby making it possible to align the balloon 3 more accurately.

The number and installation places of the markers 6 are not limited to those shown in the figure. For example, the marker can be installed at a position near the center in an axial direction of the dilation function portion 30 so that at least the position of the dilation function portion 30 can be confirmed.

The marker may confirm the position of the balloon 3 by not only fluoroscopy but also CT scanning, MRI or the like.

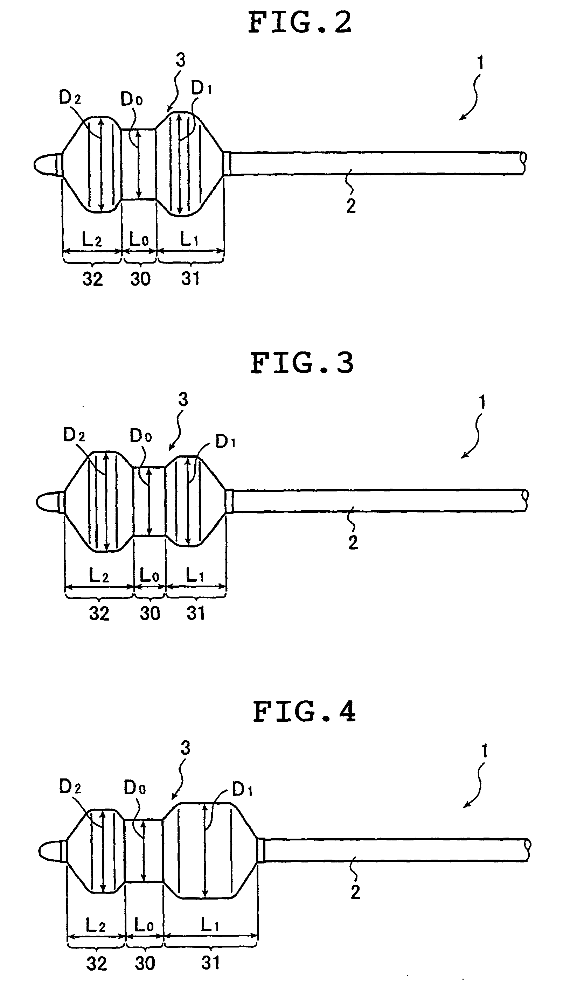

Figs. 2 to 5 are partial plan views of other embodiments of a balloon catheter comprising the balloon of the present invention. These embodiments will be described, mainly focusing on their differences from the above embodiment and the descriptions of the same items are omitted.

The balloon catheter 1 shown in Figs. 2 to 5 differs from the above balloon catheter in the shape of the balloon 3 but is the same in other elements.

The balloon 3 of the balloon catheter 1 shown in Fig. 2 is such that the maximum outer diameter $D_1$ of the first fixing portion 31 and the maximum outer diameter $D_2$ of the second fixing portion 32 under the first dilation pressure satisfy the relationship $D_1 > D_2$. In this case, the diameters $D_1$ and $D_2$ particularly preferably satisfy the relationship $D_1 > 1.1D_2$. According to this constitution, when the balloon 3 is inflated in the blood vessel, the effect of preventing the balloon 3 from moving in the distal end direction of the balloon catheter 1 is exhibited markedly.

The balloon 3 of the balloon catheter 1 shown in Fig. 3 is such that the maximum outer diameter $D_1$ of the first fixing portion 31 and the maximum outer diameter $D_2$ of the second fixing portion 32 under the first dilation pressure satisfy the relationship $D_1 < D_2$. In this case, the diameters $D_1$ and $D_2$ particularly preferably satisfy the relationship $1.1D_1 < D_2$. According to this constitution, when the balloon 3 is inflated in the blood vessel, the effect of preventing the balloon 3 from moving in the proximal end direction of the balloon catheter 1 is exhibited markedly.

The balloon 3 of the balloon catheter 1 shown in Fig. 4 is such that the length $L_1$ in the axial direction of the first fixing portion 31 and the length $L_2$ in the axial direction of the second fixing portion 32 under the first dilation pressure satisfy the relationship $L_1 > L_2$. In this case, the lengths $L_1$ and $L_2$ particularly preferably satisfy the relationship $L_1 > 1.1L_2$. According to this constitution, when the balloon 3 is inflated in the blood vessel, the effect of preventing the balloon 3 from moving in the distal end direction of the balloon catheter 1 is exhibited markedly.

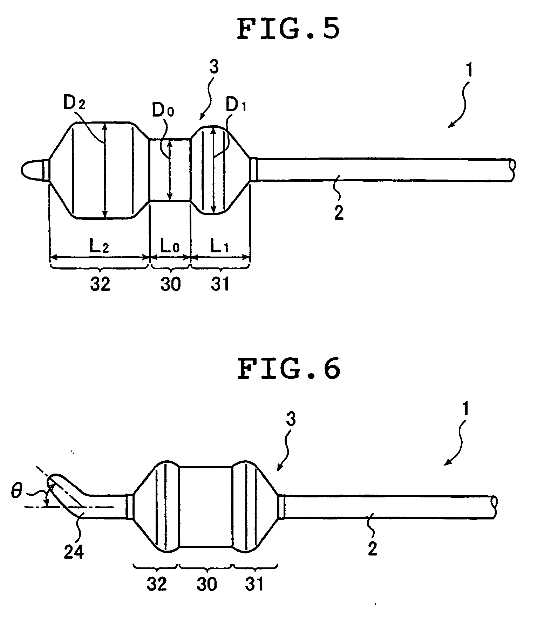

The balloon 3 of the balloon catheter 1 shown in Fig. 5 is such that the length $L_1$ in the axial direction of the first fixing portion 31 and the length $L_2$ in the axial direction of the second fixing portion 32 under the first dilation pressure satisfy the relationship $L_1 < L_2$. In this case, the lengths $L_1$ and $L_2$ particularly preferably satisfy the relationship $1.1L_1 < L_2$. According to this constitution, when the balloon 3 is inflated in the blood vessel, the effect of preventing the balloon 3 from moving in the proximal end direction of the balloon catheter 1 is exhibited markedly.

Particularly when the balloon catheter 1 is used for the PTA of a shunt formed by inosculating the ulnar artery with the basilic vein or a shunt formed by inosculating the radial artery with the cephalic vein is used, front and rear portions of a stricture may differ from each other in the inner diameter of the blood vessel, or a steep curve (bending) or branches of the blood vessel may be existent in the front or rear of the stricture. PTA suitable for the shape of the blood vessel is made possible by selecting an appropriate one from the balloon catheters 1 shown in Figs. 1 to 5, and excellent stricture dilation function and balloon fixing function can be obtained.

The shape of the balloon shown in Fig. 7 is such that the dilation function portion 30 does not have a cylindrical shape with almost a fixed outer diameter over a predetermined length unlike the shape shown in Figs. 1 to 6 but has a shape with an outer diameter increasing from a cardinal point 18 toward the first fixing portion 31 and the second fixing portion 32.

When the balloons formed as shown in Figs. 1 - 6 are inflated, the fixing portions 31, 32 having a certain length are inflated more than the dilation function portion 30 that is inflated at least to an outer diameter required for dilating the stricture. Therefore, the balloon having the shape shown in Fig. 7 can favorably provide a lower-stimulating therapy for a curved stricture having a small diameter or a stricture having a relatively short length, or when it is desired to limit the length of the blood vessel to be stimulated by inflation to a minimum.

Fig. 6 is a partial plan view of another embodiment of a balloon catheter comprising the balloon of the present invention. This embodiment will be described, mainly focusing on its differences from the above embodiments and descriptions of the same items are omitted.

The balloon catheter 1 shown in Fig. 6 is such that the catheter body 2 project from the distal end of the balloon 3 by a predetermined length (for example, about 2 to 100 mm) and the projecting portion 24 is bent at an intermediate position. This bending angle θ is not particularly limited but preferably 90° or less, more preferably 15 to 75°, much more preferably 35 to 60°.

The balloon catheter 1 having the above projecting portion 24 can easily and surely approach the blood vessel which branches off (or bends) at an acute angle like a shunt inosculating portion.

When the guide wire is inserted into the lumen 23 to project the end of the guide wire from an opening at the end of the projecting portion 24, the bending angle θ of the projecting portion 24 is reduced by the rigidity of the guide wire. When a guide wire having relatively high rigidity is used, the projecting portion 24 becomes almost as straight as an arrow.

The projecting portion 24 is not limited to the crooked shape shown in the figure but may be crooked at multiple locations in different directions.

The function and effect of the above balloon catheter 1 will be described when it is used to dilate a stricture generated near the shunt inosculating portion.

In case of dilating the stricture portion 13 generated near a shunt inosculating portion 10 formed by inosculating the vein (radial vein or cephalic vein) with the artery 11 (radial artery, etc.), since there is a difference in the inner diameter of the blood vessel between front and rear portions of the stricture 13 and the blood vessel branches off at an acute angle, when a conventional balloon catheter comprising a balloon having no difference in outer diameter at the inflation time is used, even if the balloon before inflation is located at a proper position, the balloon is moved in a front or rear direction by reaction force from the stricture and deviates from the stricture at the inflation time. Even if the balloon does not deviate from the stricture, the balloon may be fixed to the blood vessel instably and may deviate from the stricture easily.

In contrast to this, when the balloon catheter 1 of the present invention is used, the following function is obtained.

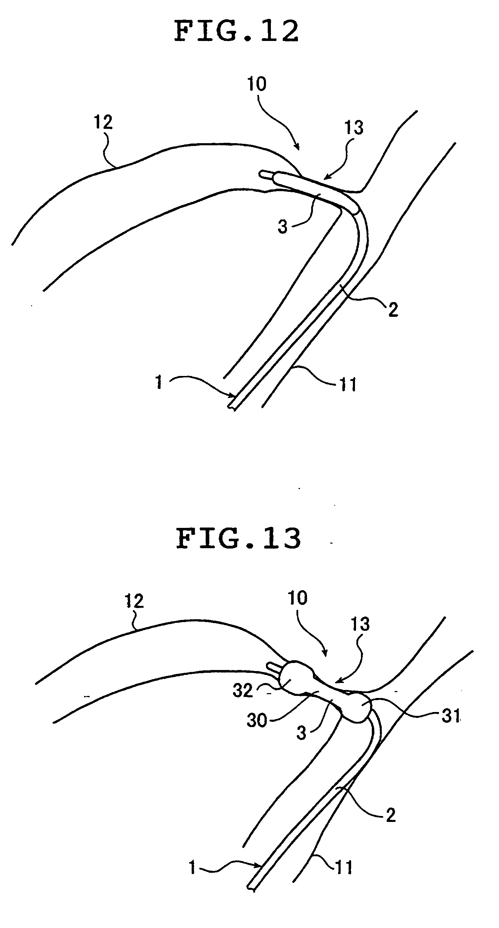

As shown in Fig. 12, the distal end portion of the catheter body 2 is guided by the guide wire inserted into the lumen 23 to position the shrunk (especially deflated and folded) balloon 3 in the stricture 13. This positioning can be carried out easily and surely by providing the marker 6.

The working fluid is injected into the balloon 3 through the lumen 21 in this state to inflate the balloon 3 gradually. Along with this, as shown in Fig. 13, the first fixing portion 31 and the second fixing portion 32 of the balloon 3 are contacted tightly to the inner surface of the blood vessel on the proximal end side and distal end side of the stricture 13, respectively, to fix the balloon 3 to the blood vessel. Since the balloon 3 is fixed at both (front and rear) ends of the stricture 13, the fixed balloon 3 does not dislocate in an axial direction. At the same time, the dilation function portion 30 of the balloon 3 is contacted tightly to the stricture 13 and dilates the stricture 13 by pressure.

When the inside pressure of the balloon 3 reaches the first dilation pressure, the outer diameter of each part of the balloon 3 is rarely increased by further raising the inside pressure of the balloon 3. Thus, it is possible to dilate the stricture 13 properly and safely without dilating the stricture 13 excessively, and a required and satisfactory treatment effect can be expected.

Since the balloon 3 is molded such that it achieves a predetermined shape when it is inflated, a desired balloon 3 is selected to easily control the required expanded diameter of the stricture 13 and proper PTA can be realized. The location of the stricture 13, the degree of stricture and an expanded diameter required for the stricture can be known in advance by injecting a contrast medium into a portion near the stricture 13. Therefore, a balloon 3 suitable for these conditions can be easily selected.

Thus, in the present invention, it is not necessary to carry out re-dilation caused by the deviation from the stricture or dislocation of the balloon and to carry out under-dilation in order to avoid deviation or dislocation, thereby making it possible to carry out PTA without costing much labor in a shorter period of time and to reduce the burden of a patient.

When it is judged that the inflation state of the balloon 3 is maintained for a predetermined time and the stricture 13 is fully dilated (a satisfactory blood passage is secured), the working fluid in the balloon 3 is removed through the lumen 21, the balloon 3 is deflated again, and the balloon catheter 1 is removed from the blood vessel. Thus, PTA is completed.

The balloon for dilating a stricture of the present invention and a balloon catheter comprising the same have been described with reference to embodiments shown in the figures. However, the present invention is not limited thereto.

The balloon catheter of the present invention can be inserted into a lumen other than the blood vessel, for example, a digestive canal, trachea, bile duct, urethra, ureter or the like.

As having been described above, according to the present invention, the balloon can be fixed to the stricture without fail when the balloon is inflated and the stricture can be dilated properly and safely.

It is not necessary to carry out re-dilation caused by the deviation etc. from the stricture of the balloon or under-dilation in order to avoid deviation or the like, thereby making it possible to easily carry out PTA in a shorter period of time and to reduce the burden of a patient.

Particularly when a stricture generated at a location where the inner diameter of the blood vessel changes or the blood vessel bends or branches off at an acute angle like a shunt inosculating portion is to be dilated, this effect is exhibited more effectively.

When the balloon is produced by biaxial orientation blow molding, the balloon can be produced with high yield and a balloon having small fluctuations in shape, film strength, characteristics, etc. can be easily produced.

## Claims

1. A balloon (3) for dilating a stricture (stenosis) which can be inflated and deflated by a change in inside pressure and comprises a dilation function portion (30) having the function to contact tightly to the stricture portion of a lumen and dilating the stricture by pressure, and a first fixing portion (31) and a second fixing portion (32) located on proximal end and distal end sides of the dilation function portion (30), respectively and having the function to fix the balloon (3) to the lumen,

   characterized in that when the minimum outer diameter of the dilation function portion (30) under a first dilation pressure at which the dilation function portion (30) dilates to a predetermined diameter is represented by $D_0$, the minimum outer diameter of the dilation function portion (30) under a second dilation pressure at which the balloon (3) ruptures by a rise in inside pressure is represented by $D_0max$, the maximum outer diameter of the first fixing portion (31) is represented by $D_1$ and the maximum outer diameter of the second fixing portion is represented by $D_2$, the balloon (3) is molded to satisfy the following formulas /1/ and /2/ under the first dilation pressure and to satisfy the following formulas /3/ and /4/ under the second dilation pressure:

$$D_0 < D_1 \qquad /1/,$$

$$D_0 < D_2 \qquad /2/,$$

$$D_0max < D_1 \qquad /3/,$$

and

$$D_0max < D_2 \qquad /4/.$$

2. The balloon (3) for dilating a stricture according to claim 1, which satisfies $D_0max - D_0 \leq 0.15D_0$ wherein the minimum outer diameter of the dilation function portion (30) under second dilation pressure at which the balloon (3) ruptures by a rise in inside pressure is represented by $D_0max$.

3. The balloon (3) for dilating a stricture according to claim 1 or 2, which satisfies $D_1 > D_2$ under the first dilation pressure.

4. The balloon (3) for dilating a stricture according to claim 1 or 2, which satisfies $D_1 < D_2$ under the first dilation pressure.

5. The balloon (3) for dilating a stricture according to any one of claims 1 to 4, which satisfies $0.02D_0 \leq \Delta D \leq 0.4D_0$, wherein $\Delta D = (D_1 + D_2)/2 - D_0$.

6. The balloon (3) for dilating a stricture according to any one of claims 1 to 5, wherein $\Delta D$ is 0.02 to 20

mm, wherein $\Delta D = (D_1 + D_2)/2 - D_0$.

7. The balloon (3) for dilating a stricture according to any one of claims 1 to 6, wherein $L_0$ is 5 to 50 mm, wherein the length in an axial direction of the dilation function portion (30) under the first dilation pressure is represented by $L_0$.

8. The balloon (3) for dilating a stricture according to any one of claims 1 to 7, which satisfies $L_1 > L_2$ wherein the length in an axial direction of the first fixing portion (31) is represented by $L_1$ and the length in an axial direction of the second fixing portion (32) is represented by $L_2$ under the first dilation pressure.

9. The balloon (3) for dilating a stricture according to any one of claims 1 to 7, which satisfies $L_1 < L_2$ wherein the length in an axial direction of the first fixing portion (31) is represented by $L_1$ and the length in an axial direction of the second fixing portion (32) is represented by $L_2$ under the first dilation pressure.

10. The balloon (3) or dilating a stricture according to any one of claims 1 to 9, which is produced by biaxial orientation and blow molding.

11. The balloon (3) or dilating a stricture according to any one of claims 1 to 10, wherein the first fixing portion (31) and/or the second fixing portion (32) have/has higher pressure resistance than the dilation function portion (30).

12. A balloon catheter (1) comprising a catheter body (2) having flexibility and the balloon (3) for dilating a stricture according to any one of claims 1 to 11 at an end of the catheter body (2).

13. The balloon catheter (1) according to claim 12, wherein a lumen (21) which is the passage of a working fluid for inflating and deflating the balloon (3) for dilating a stricture and a lumen (23) for containing a guide wire are formed in the catheter body (2).

14. The balloon cathete (1) according to claim 12 or 13, which comprises a marker (6) or confirming the position of the balloon (3) for dilating a stricture in a body.

15. The balloon catheter (1) according to claim 14, wherein at least the position of the dilation function portion (30) can be confirmed with the marker (6).

16. The balloon catheter (1) according to any one of claims 12 to 15, wherein the catheter body (2) projects from the distal end of the balloon (3) for dilating a stricture by a predetermined length and the projecting portion (24) bends at least one time.

17. The balloon catheter (1) according to any one of claims 1 to 16, wherein the dilation function portion (30) has a constant predetermined outer diameter ($D_0$) along its axial length ($L_0$).

18. The balloon catheter (1) according to any one of claims 1 to 16, wherein the dilation function portion (30) has a minimum outer diameter in the center of its axial length (at 18), and the outer diameter continuously increases toward both of the first and the second fixing portion (31, 32).

**Patentansprüche**

1. Ein Ballon (3) zum Dilatieren einer Verengung (Stenosis), der durch eine Veränderung des innenseitigen Drucks aufgeblasen und entleert werden kann und umfasst: einen Dilatationsfunktionsbereich (30) mit der Funktion, den verengten Bereich eines Lumens fest zu berühren und die Verengung durch Druck zu dilatieren, sowie einen ersten Befestigungsbereich (31) bzw. einen zweiten Befestigungsbereich (32), die auf der Seite des proximalen Endes bzw. distalen Endes des Dilatationsfunktionsbereichs (30) angeordnet sind und die Funktion aufweisen, den Ballon (3) an dem Lumen zu befestigen, **dadurch gekennzeichnet, dass** wenn der minimale äußere Durchmesser des Dilatationsfunktionsbereichs (30) bei einem ersten Dilatationsdruck, bei dem der Dilatationsfunktionsbereich (30) sich auf einen vorbestimmten Durchmesser erweitert, mit $D_0$ dargestellt wird, der minimale äußere Durchmesser des Dilatationsfunktionsbereichs (30) bei einem zweiten Dilatationsdruck, bei dem der Ballon (3) durch einen Anstieg des innenseitigen Drucks zerreißt, durch $D_0^{max}$ dargestellt wird, der maximale äußere Durchmesser des ersten Befestigungsbereichs (31) durch $D_1$ dargestellt wird und der maximale äußere Durchmesser des zweiten Befestigungsbereichs (32) durch $D_2$ dargestellt wird, der Ballon (3) so geformt wird, das er die folgenden Gleichungen /1/ und /2/ bei dem ersten Dilatationsdruck erfüllt und die folgenden Gleichungen /3/ und /4/ bei dem zweiten Dilatationsdruck erfüllt:

$$D_0 < D_1 \qquad\qquad /1/,$$

$$D_0 < D_2 \qquad\qquad /2/,$$

$$D_0\max < D_1 \qquad /3/,$$

und

$$D_0\max < D_2 \qquad /4/.$$

**2.** Der Ballon (3) zum Dilatieren einer Verengung nach dem Anspruch 1, der $D_0\max - D_0 < 0{,}15\,D_0$ erfüllt, wobei der minimale äußere Durchmesser des Dilatationsfunktionsbereichs (30) beim zweiten Dilatationsdruck, bei dem der Ballon (3) durch einen Anstieg des innenseitigen Drucks zerreißt, durch $D_0\max$ dargestellt wird.

**3.** Der Ballon (3) zum Dilatieren einer Verengung nach dem Anspruch 1 oder 2, der bei dem ersten Dilatationsdruck $D_1 > D_2$ erfüllt.

**4.** Der Ballon (3) zum Dilatieren einer Verengung nach dem Anspruch 1 oder 2, der bei dem ersten Dilatationsdruck $D_1 < D_2$ erfüllt.

**5.** Der Ballon (3) zum Dilatieren einer Verengung nach einem der Ansprüche 1 bis 4, der $0{,}02D_0 < \not{c}D < 0{,}4D_0$ erfüllt, wobei $\not{c}D = (D_1 + D_2)/2 - D_0$.

**6.** Der Ballon (3) zum Dilatieren einer Verengung nach einem der Ansprüche 1 bis 5, wobei $\not{c}D$ 0,02 bis 20 mm ist, wobei $\not{c}D = (D_1 + D_2)/2 - D_0$.

**7.** Der Ballon (3) zum Dilatieren einer Verengung nach einem der Ansprüche 1 bis 6, wobei $L_0$ 5 bis 50 mm ist, wobei die Länge in einer axialen Richtung des Dilatationsfunktionsbereichs (30) bei dem ersten Dilatationsdruck durch $L_0$ dargestellt wird.

**8.** Der Ballon (3) zum Dilatieren einer Verengung nach einem der Ansprüche 1 bis 7, der bei dem ersten Dilatationsdruck $L_1 > L_2$ erfüllt, wobei die Länge in einer axialen Richtung des ersten Befestigungsbereichs (31) durch $L_1$ dargestellt wird und die Länge in einer axialen Richtung des zweiten Befestigungsbereichs (32) durch $L_2$ dargestellt wird.

**9.** Der Ballon (3) zum Dilatieren einer Verengung nach irgend einem der Ansprüche 1 bis 7, der bei dem ersten Dilatationsdruck $L_1 < L_2$ erfüllt, wobei die Länge in einer axialen Richtung des ersten Befestigungsbereichs (31) durch $L_1$ dargestellt wird und die Länge in einer axialen Richtung des zweiten Befestigungsbereichs (32) durch $L_2$ dargestellt wird.

**10.** Der Ballon (3) zum Dilatieren einer Verengung nach einem der Ansprüche 1 bis 9, der durch biaxiale Ausrichtung und Blasformen hergestellt wird.

**11.** Der Ballon (3) zum Dilatieren einer Verengung nach einem der Ansprüche 1 bis 10, wobei der erste Befestigungsbereich (31) und/oder der zweite Befestigungsbereich (32) eine höhere Druckwiderstandsfähigkeit aufweisen als der Dilatationsfunktionsbereich (30).

**12.** Ein Ballonkatheter (1) mit einem Flexibilität aufweisenden Katheterteil (2) und dem an einem Ende des Katheterteils (2) angeordneten Ballon (3) zum Dilatieren einer Verengung nach einem der Ansprüche 1 bis 11.

**13.** Das Ballonkatheter (1) nach Anspruch 12, wobei in dem Katheterteil (2) ein Lumen (21), welches der Durchlaß einer Arbeitsflüssigkeit zum Aufblasen und Entleeren des Ballons (3) zum Dilatieren einer Verengung ist, und ein Lumen (23) zum Aufnehmen eines Führungsdrahts gebildet sind.

**14.** Das Ballonkatheter (1) nach Anspruch 12 oder 13, das eine Markierung (6) zum Bestätigen der Position des Ballons (3) zum Dilatieren einer Verengung in einem Körper umfasst.

**15.** Das Ballonkatheter (1) nach Anspruch 14, wobei zumindest die Position des Dilatationsfunktionsbereichs (30) mit der Markierung (6) bestätigt werden kann.

**16.** Das Ballonkatheter (1) nach einem der Ansprüche 12 bis 15, wobei das Katheterteil (2) von dem distalen Ende des Ballons (3) zum Dilatieren einer Verengung um eine vorbestimmte Länge herausragt und der herausragende Teil (24) zumindest einmal gekrümmt ist.

**17.** Das Ballonkatheter (1) nach einem der Ansprüche 1 bis 16, wobei der Dilatationsfunktionsbereich (30) entlang seiner axialen Länge ($L_0$) einen konstanten, vorbestimmten äußeren Durchmesser ($D_0$) aufweist.

**18.** Das Ballonkatheter (1) nach einem der Ansprüche 1 bis 16, wobei der Dilatationsfunktionsbereich (30) in der Mitte seiner axialen Länge (bei 18) einen minimalen äußeren Durchmesser aufweist und der äußere Durchmesser in Richtung sowohl zum ersten als auch zum zweiten Befestigungsbereich (31, 32) hin kontinuierlich zunimmt.

**Revendications**

**1.** Ballonnet (3) pour dilater un rétrécissement (sténose), qui peut être gonflé et dégonflé par un changement de pression intérieure et qui comprend une partie à fonction de dilatation (30) ayant pour fonction de venir en contact étroit avec la partie de rétrécissement d'une lumière et de dilater le rétrécissement par pression, et une première partie de fixation (31) et une deuxième partie de fixation (32) placées sur les cotés d'extrémité proximale et d'extrémité distale de la partie à fonction de dilatation (30), respectivement et ayant pour fonction de fixer le ballonnet (3) à la lumière,

**caractérisé en ce que** quand le diamètre extérieur minimum de la partie à fonction de dilatation (30) sous une première pression de dilatation à laquelle la partie à fonction de dilatation (30) se dilate à un diamètre prédéterminé est représenté par $D_0$, le diamètre extérieur minimum de la partie à fonction de dilatation (30) sous une deuxième pression de dilatation à laquelle le ballonnet (3) se rompt par une augmentation de la pression intérieure est représenté par $D_0max$, le diamètre extérieur maximum de la première partie de fixation (31) est représenté par $D_1$ et le diamètre extérieur maximum de la deuxième partie de fixation (32) est représenté par $D_2$, le ballonnet (3) est moulé pour répondre aux formules suivantes /1/ et /2/ sous la première pression de dilatation et pour répondre aux formules suivantes /3/ et /4/ sous la deuxième pression de dilatation :

$$D_0 < D_1 \qquad /1/,$$

$$D_0 < D_2 \qquad /2/,$$

$$D_0max < D_1 \qquad /3/,$$

et

$$D_0max < D_2 \qquad /4/.$$

2. Ballonnet (3) pour dilater un rétrécissement selon la revendication 1, qui répond à $D_0max - D_0 \leq 0,15D_0$, dans lequel le diamètre extérieur minimum de la partie à fonction de dilatation (30) sous une deuxième pression de dilatation à laquelle le ballonnet (3) se rompt par une augmentation de la pression intérieure est représenté par $D_0max$.

3. Ballonnet (3) pour dilater un rétrécissement selon la revendication 1 ou 2, qui répond à $D_1 > D_2$ sous la première pression de dilatation.

4. Ballonnet (3) pour dilater un rétrécissement selon la revendication 1 ou 2, qui répond à $D_1 < D_2$ sous la première pression de dilatation.

5. Ballonnet (3) pour dilater un rétrécissement selon l'une quelconque des revendications 1 à 4, qui répond à $0,02D_0 \leq \Delta D \leq 0,4 D_0$ où $\Delta D = (D_1 + D_2)/2 - D_0$.

6. Ballonnet (3) pour dilater un rétrécissement selon l'une quelconque des revendications 1 à 5, où $\Delta D$ va de 0,02 à 20 mm, où $\Delta D = (D_1 + D_2)/2 - D_0$.

7. Ballonnet (3) pour dilater un rétrécissement selon l'une quelconque des revendications 1 à 6, dans lequel L0 va de 5 à 50 mm, où la longueur dans une direction axiale de la partie à fonction de dilatation (30) sous la première pression de dilatation est représentée par $L_0$.

8. Ballonnet (3) pour dilater un rétrécissement selon l'une quelconque des revendications 1 à 7, qui répond à $L_1 > L_2$, où la longueur dans une direction axiale de la première partie de fixation (31) est représentée par $L_1$ et la longueur dans une direction axiale de la deuxième partie de fixation (32) est représentée par $L_2$ sous la première pression de dilatation.

9. Ballonnet (3) pour dilater un rétrécissement selon l'une quelconque des revendications 1 à 7, qui répond à $L_1 < L_2$, où la longueur dans une direction axiale de la première partie de fixation (31) est représentée par $L_1$ et la longueur dans une direction axiale de la deuxième partie de fixation (32) est représentée par $L_2$ sous la première pression de dilatation.

10. Ballonnet (3) pour dilater un rétrécissement selon l'une quelconque des revendications 1 à 9, qui est produit par orientation biaxiale et par soufflage.

11. Ballonnet (3) pour dilater un rétrécissement selon l'une quelconque des revendications 1 à 10, dans lequel la première partie de fixation (31) et/ou la deuxième partie de fixation (32) ont/a une résistance à la pression supérieure à celle de la partie à fonction de dilatation (30).

12. Cathéter à ballonnet (1) comprenant un corps de cathéter (2) présentant une flexibilité et le ballonnet (3) pour dilater un rétrécissement selon l'une quelconque des revendications 1 à 11 à une extrémité du corps de cathéter (2).

13. Cathéter à ballonnet (1) selon la revendication 12, dans lequel une lumière (21) qui est le passage d'un fluide de travail pour gonfler et dégonfler le ballonnet (3) pour dilater un rétrécissement et une lumière (23) pour contenir un fil de guidage sont formées dans le corps de cathéter (2).

**14.** Cathéter à ballonnet (1) selon la revendication 12 ou 13, qui comprend un repère (6) pour confirmer la position du ballonnet (3) pour dilater un rétrécissement d'un corps.

**15.** Cathéter à ballonnet (1) selon la revendication 14, dans lequel au moins la position de la partie à fonction de dilatation (30) peut être confirmée à l'aide du repère (6).

**16.** Cathéter à ballonnet (1) selon l'une quelconque des revendications 12 à 15, dans lequel le corps de cathéter (2) dépasse de l'extrémité distale du ballonnet (3) pour dilater un rétrécissement d'une longueur prédéterminée et la partie saillante (24) fléchit au moins une fois.

**17.** Cathéter à ballonnet (1) selon l'une quelconque des revendications 1 à 16, dans lequel la partie à fonction de dilatation (30) a un diamètre extérieur prédéterminé constant ($D_0$) sur sa longueur axiale ($L_0$).

**18.** Cathéter à ballonnet (1) selon l'une quelconque des revendications 1 à 16, dans lequel la partie à fonction de dilatation (30) a un diamètre extérieur minimum au milieu de sa longueur axiale en 18, et le diamètre extérieur augmente de façon continue vers la première et la deuxième partie de fixation (31, 32).

# FIG.1

# FIG.2

# FIG.3

# FIG.4

# FIG.5

# FIG.6

# FIG.7

EP 1 062 966 B1

# FIG.8

# FIG.9

# FIG.10

# FIG.11

# FIG.12

# FIG.13